# EUROPEAN PATENT SPECIFICATION

(11) **EP 1 595 573 B1**
(45) Date of publication and mention of the grant of the patent: **01.10.2008**
(21) Application number: 05018035.5
(22) Date of filing: 13.08.1997
(51) Int. Cl.: A61K 31/00, A61K 31/255, A61L 31/16, A61P 9/10, A61K 31/225, A61K 31/198, A61K 31/4192, A61K 31/4196, A61K 31/315, A61K 31/32

(54) **Stent comprising manipulators of nitrosative stress to reduce restenosis**
Stent, welcher Verbindungen enthält, die nitrosativen Stress beeinflussen, gegen Restenose
Stent contenant des manipulateurs du stress nitrosatif pour le traitement de la restenose

(30) Priority: 30.08.1996 US 25819 P; 07.05.1997 US 852490
(43) Date of publication of application: 16.11.2005
(62) Divisional of application: 97938149.8
(73) Proprietor: Duke University, Durham, NC 27708-0083 (US); THE MEDICAL COLLEGE OF WISCONSIN RESEARCH FOUNDATION, INC., Milwaukee, WI 53226 (US)
(72) Inventor: Stamler, Jonathan S., Chapel Hill, NC 27514 (US); Griffith, Owen W., Milwaukee, WI 53210 (US)
(74) Representative: Burford, Anthony Frederick

(56) References cited:
- WO-A-96/33757
- US-A- 5 676 963
- MYUNG HO JEONG ET AL: "Biological and genetic therapy for restenosis" CHONNAM JOURNAL OF MEDICAL SCIENCES, RESEARCH INSTITUTE OF MEDICAL SCIENCES. CHONNAM NATIONAL UNIVE, KR, vol. 9, no. 1, 1996, pages 122-129, XP002957971 ISSN: 1013-3968
- FOLTS JOHN D ET AL: "Palmaz-Schatz stents coated with a NO donor reduces reocclusion when placed in pig carotid arteries for 28 days" JOURNAL OF THE AMERICAN COLLEGE OF CARDIOLOGY, vol. 27, no. 2 SUPPL. A, 1996, page 86A, XP008054479 & 45TH ANNUAL SCIENTIFIC SESSION OF THE AMERICAN COLLEGE OF CARDIOLOGY; ORLANDO, FLORIDA, USA; MARCH 24-27, 1996 ISSN: 0735-1097
- DENNINGER M-H ET AL: "Budd-Chiari syndrome and factor v Leiden mutation [20]" LANCET 1995 UNITED KINGDOM, vol. 345, no. 8948, 1995, pages 525-526, XP008054470 ISSN: 0140-6736
- FOLTS JOHN D ET AL: "Coating Palmaz-Schatz stents with a unique NO donor renders them much less thrombogenic when placed in pig carotid arteries" CIRCULATION, vol. 92, no. 8 SUPPL., 1995, page I670, XP008054483 & 68TH SCIENTIFIC SESSION OF THE AMERICAN HEART ASSOCIATION; ANAHEIM, CALIFORNIA, USA; NOVEMBER 13-16, 1995 ISSN: 0009-7322
- KAPLAN BARRY M ET AL: "Prospective study of intracoronary verapamil and nitroglycerin for the treatment of no-reflow after interventions on degenerated saphenous vein grafts" CIRCULATION, vol. 92, no. 8 SUPPL., 1995, page I330, XP008054476 & 68TH SCIENTIFIC SESSION OF THE AMERICAN HEART ASSOCIATION; ANAHEIM, CALIFORNIA, USA; NOVEMBER 13-16, 1995 ISSN: 0009-7322
- KAUL SANJAY ET AL: "Inhibition of acute stent thrombosis under high-shear flow conditions by a nitric oxide donor, DMHD/NO: An ex vivo porcine arteriovenous shunt study" CIRCULATION, vol. 94, no. 9, 1996, pages 2228-2234, XP008054482 ISSN: 0009-7322
- MELLIERE D ET AL: "[Antibiotic-impregnated prostheses: eclectic indications]" JOURNAL DES MALADIES VASCULAIRES. 1996, vol. 21 Suppl A, 1996, pages 139-145, XP008054473 ISSN: 0398-0499

## Description

This invention was made at least in part with US Government support under National Institutes of Health Grant Nos. DK48423, HL02582 and HL52529. The US Government has certain rights in the invention.

### TECHNICAL FIELD

The invention herein is directed to a stent for treating patients to selectively kill or reduce the growth of pathologically proliferating mammalian cells that would cause restenosis

### BACKGROUND OF THE INVENTION

Nitric oxide (NO) is now recognized as a signaling molecule in biology and has been implicated in the function of virtually every organ system in mammals. It is known that NO relaxes blood vessels, intestines, airways and skeletal muscles and plays a role in memory, sexual behavior and host defense. On the other hand, excessive production of NO has been implicated in organ dysfunction, degenerative disease and promotion of cancer. Arthritis, ulcerative colitis, Alzheimer's disease, congestive heart failure, septic shock and atherosclerosis are disorders in which NO may play a pathogenic role.

It is recognized that there is normally a certain amount of endogenously produced oxidative stress in mammals, i.e., the endogenous production of reactive oxygen species in the body, e.g., superoxide, hydrogen peroxide, hydroxyl radical, hypochlorous acid and singlet oxygen. This is thought to contribute to ageing, rheumatism, atherosclerosis, inflammation, respiratory distress syndrome, fibrosis, and development of infectious diseases such as AIDS.

Before the discovery leading to the invention herein, it was not recognized that there is a nitrosative stress distinct from oxidative stress that affects mammals and also microorganisms which can be manipulated in a therapeutically effective manner by decreasing pathologically proliferating mammalian cell defenses against nitrosative stress.

### SUMMARY OF THE INVENTION

The present invention provides a stent comprising a manipulator of nitrosative stress according to the list of claim 1 which inhibits protection against nitrosative stress to selectively kill or reduce the growth of pathologically proliferating cells that would cause restenosis.

The term "nitric oxide related compounds" is used herein to mean compounds able to transfer NO⁺, NO⁻ or NO₂⁺ group to biological molecules. The term does not include nitric oxide itself. Nitric oxide itself is not a nitrosative stress agent.

The term "nitrosativen stress" is used herein to mean an impetus for NO or NO₂ group attachment to proteins, nucleic acids or other biological molecules. It may be potentially therapeutic if pathologically proliferating mammalian cells are affected or potentially pathologic if normal mammalian cells are damaged. Nitrosative stress is distinct from oxidative stress and can occur under anaerobic conditions.

It has further been discovered that to prevent damage from nitrosative stress, cells exhibit constitutive defenses as well as an adaptive response that applies to mammalian cells including human cells. An important aspect of this adaptive response is that it is distinct in its regulation and in its molecular purpose from the adaptive response to oxidative stress. Specifically, it has been discovered that cells upregulate resistance genes and other biochemical pathways to protect themselves from nitrosative stress. Thiols (e.g., glutathione in mammals L-homocysteine, mycothiol, ovothiols, etc.) and enzymes which mediate constitutive thiol synthesis comprise the first line of defense. Antinitrosative stress genes and their products comprise a second line of defense.

The term "antinitrosative stress gene" is used herein to mean a gene coding for a product that when expressed either breaks down or eliminates nitrosants (nitrosating species), denitrosates nitrosatively inhibited proteins or other biological molecules to restore their function, or upregulates other products or pathways which are protective against nitrosative stress.

The term "selectively kills or reduces the growth of mammalian cells" as used herein means kills or reduces growth of pathologically proliferating mammalian cells without causing unacceptable killing or inhibition of growth of normal mammalian cells or kills or reduces growth of pathologically proliferating mammalian cells in a percentage which is at least 10% greater than the percentage of normal mammalian cells adversely affected.

The manipulator(s) of nitrosative stress in pathologically proliferating mammalian cells that would cause restenosis comprise(s) an inhibitor of protection against nitrosative stress according to claim 1. One or more of these inhibitors of protection against nitrosative stress can be used as the only kind of manipulator of nitrosative stress or two kinds of manipulators of nitrosative stress are employed where one kind of manipulator of nitrosative stress is inhibitor of protection against nitrosative stress according to claim 1 and the other kind of manipulator of nitrosative stress is agent which increases nitrosative stress. Said inhibitor(s) is (are) employed to selectively kill or reduce the growth of said cells or to enhance their susceptibility to antiproliferation agents, i.e., to anti-restenosis drugs that function by a mechanism other than by manipulating nitrosative stress.

It is known in the prior art to insert NO releasing stents during angioplasty. However, it is not known in the prior art to administer manipulator of nitrosative stress which inhibits protection against nitrosative stress to selectively kill or reduce the growth of pathologically proliferating cells that would cause restenosis or to selectively enhance their susceptibility to antiproliferation agents that function by a mechanism other than by manipulating nitrosative stress. Moreover, it is not known in the prior art to concurrently administer manipulator of nitrosative stress which increases nitrosative stress and manipulator of nitrosative stress which inhibits protection against nitrosative stress in the pathologically proliferating cells that would cause restenosis.

The manipulator of nitrosative stress used in the invention is a selective inhibitor of protection against nitrosative stress in the pathologically proliferating mammalian cells. The term "selective inhibitor" is used in the prior sentence to refer to agent which in use downregulates nitrosative stress defense mechanism in pathologically proliferating mammalian cells so as to effect or mediate the selective killing or growth reduction recited above.

One kind of selective inhibitor of protection against nitrosative stress is a selective inhibitor of thiol synthesis or selective depleter of thiol in pathologically proliferating mammalian cells. The term "selective inhibitor of thiol synthesis" as used herein refers to selective application or action of an inhibitor of thiol synthesis which is itself not selective for inhibition of thiol synthesis in pathologically proliferating mammalian cells but which achieves selective result because of coaction with drug that has more specific application or action or by virtue of local delivery. The term "selective depleter of thiol" as used herein means agents which conjugate with thiols; agents transferring nitroso (-NO) or nitro (-NO₂) groups to thiols which, when administered systemically to the mammal, are selective for pathologically proliferating mammalian cells.

Another kind of selective inhibitor of protection against nitrosative stress is a selective inhibitor of transcription or translation of an antinitrosative stress gene (as defined above) or a selective inhibitor of an antinitrosative stress gene product.

In another aspect of the invention herein two kinds of manipulators of nitrosative stress in said pathologically proliferating mammalian cells are employed and one kind is a selective inhibitor of protection against nitrosative stress in said pathologically proliferating mammalian cells, and the other kind is a selective increaser of nitrosative stress in said pathologically proliferating mammalian cells, or prodrug therefor, and either the inhibition of protection against nitrosative stress or the increase of nitrosative stress or both are selectively effected in said mammalian cells.

One or a plurality of kinds of the manipulators of nitrosative stress can be fastened to a stent inserted during angioplasty. This provides local application of the manipulator(s) of nitrosative stress to pathologically proliferating cells that would cause restenosis. In an embodiment of the invention herein, a plurality of agents are attached to a stent to perform both the function of inhibiting glutathione synthesis in the pathologically proliferating cells and the function of increasing nitrosative stress in the pathologically proliferating cells. In one example of this method, inhibitor of thiol synthesis or depleter of thiol is attached to and administered from a stent as the sole manipulator of nitrosative stress and the insertion of the stent with inhibitor of thiol synthesis or depleter of thiol thereon is optionally followed by administration of intravascular beta-, gamma- or X-ray radiation. In a preferred method herein two kinds of manipulators of nitrosative stress are administered and both are associated with a porous polymer coated on a stent whereby the two kinds of manipulators of nitrosative stress leach out of the porous polymer and contact the pathologically proliferating cells and one kind of manipulator of nitrosative stress is L-buthionine-S-sulfoximine ("BSO") and the other kind of manipulator of nitrosative stress is an agent that increases nitrosative stress. In an alternative, L-buthionine-S-sulfoximine is fastened to and administered from a stent and agent that increases nitrosative stress is administered systemically.

### DETAILED DESCRIPTION

The present invention provides a stent comprising a manipulator of nitrosative stress which inhibits protection against nitrosative stress to selectively kill or reduce the growth of pathologically proliferating cells that would cause restenosis, as defined in claim 1.

The selective inhibitors of protection against nitrosative stress in the pathologically proliferating mammalian cells as defined in claim 1 include, for example, selective inhibitors of thiol synthesis or selective depleters of thiol in the pathologically proliferating mammalian cells, selective inhibitors of transcription or translation of an antinitrosative stress gene in the pathologically proliferating mammalian cells and selective inhibitors of an antinitrosative stress gene product in the pathologically proliferating mammalian cells.

We now turn to cases where the manipulator of nitrosative stress is a selective inhibitor of protection against nitrosative stress in the pathologically proliferating mammalian cells is a selective inhibitor of thiol synthesis or selective depleter of thiol in the pathologically proliferating mammalian cells. The terms "selective inhibitor of thiol synthesis" and "selective depleter of thiol" apply to agents which selectively inhibit protection against nitrosative stress in pathologically proliferating mammalian cells, with selectivity occurring because of specific effect either (a) because of greater effect on pathologically proliferating mammalian cells than on other cells either because of more specific application or action or because of coaction with drug that has more specific application or action, or (b) because of local delivery.

Glutathione protects against nitrosative stress in mammals. Thus, selective inhibitors of glutathione synthesis and selective depleters of glutathione falling under present claim 1, are administered herein to inhibit growth of pathologically proliferating mammalian cells in mammals.

Inhibitors of glutathione synthesis for use in inhibiting proliferation of pathologically proliferating cells that would cause restenosis include, for example, non-selective inhibitor of glutathione synthesis, e.g., L-buthionine-S-sulfoximine attached to a stent, optionally used in combination with administration of intravascular beta-, gamma- or X-ray radiation. Specific embodiments include L-buthionine-S-sulfoximine attached to a polymer stent by ester linkage in which case it is released by extracellular esterases, or incorporating L-buthionine-S-sulfoximine into a porous polymer stent from which it slowly diffuses once the stent has been placed in the occluded artery. In these cases, appropriate dosing requires that the stent incorporate 0.1 to 10% L-buthionine-S-sulfoximine by weight of the polymer used in the stent. The route of administration is local. Intravascular beta- or gamma-radiation is optionally applied, for example, by using a Palaz-Schatz Stent implanted with radioactive ³²P, e.g., at a dosage of 0. 1µCi, or ¹⁹²Ir, e.g., at a dosage ranging from 350-2500cGy, preferably at a dosage greater than 1400cGy, or ⁹⁰Sr/Y, e.g., at a dosage ranging from 7-56Gy.

Depleters of glutathione for use in inhibiting proliferation of pathologically proliferating cells that would cause restenosis include, for example, ethyl maleate attached to polymer stent by ester linkage or ethyl maleate agent incorporated into a porous polymer stent such that it slowly diffuses from the polymer stent emplaced in an occluded artery. Nitrosating agents attached to a polymer stent or incorporated into a porous polymer stent contribute both to thiol depletion and to nitrosative stress. For all these drugs, appropriate dosages are 0.1 - 10% by weight of polymer used in the stent. The route of administration is local.

For inhibiting proliferation of pathologically proliferating cells that would cause restenosis, the rate of drug release should be such that the duration of therapy following implantation of the stent is 1 to 20 days or longer.

Antitumor alkylator, e.g., melphalan, can be administered locally as a mechanism of glutathione depletion in proliferating cells, preferably with co-administration locally of agent that inhibits the synthesis of glutathione, e.g., L-buthionine-S-sulfoximine, with or without co-administration of agent that increases nitrosative stress. For example, the alkylator is associated with polymer in the stent and the alkylator is present in an amount of 0.1 to 10% by weight of polymer in the stent and drug release is such that the therapy continues for 1 to 20 days after insertion of the stent.

For inhibiting the proliferation of pathologically proliferating cells that would cause restenosis, the agents are made selective by attachment to a polymer stent or by incorporation thereof in a porous polymer stent in an amount ranging from 0.1 to 10% by weight of the polymer in the stent and the rate of drug release should be such that the duration of drug therapy following implantation of the stent is 1 to 20 days or longer.

We turn now to the cases where a selective inhibitor of an antinitrosative stress gene product falling under claim 1 is administered to a mammal to inhibit the proliferation (growth) of pathologically proliferating mammalian cells in the mammal. The inhibitor can be, for example, aminotriazole which inhibits catalase. Catalase has as a side activity, the ability to convert NO₂⁻ to NO₃⁻. Once activated by H₂O₂, NO₂⁻ is a much stronger nitrosating agent than NO₃⁻. Thus, inhibition of catalase results in stronger nitrosating agent being present. Aminotriazole is made selective by attachment (e.g., by amide linkage) to polymer of a polymer stent or by incorporation into a porous polymer stent in an amount ranging from 0.1 to 10% by weight of the polymer in the stent and the rate of drug release should be such that the duration of drug therapy following implantation of the stent is 1 to 20 days or longer.

Another example of an inhibitor of an antinitrosative stress gene product for administration to a mammal to inhibit the proliferation (growth) of pathologically proliferating cells in the mammal are the inhibitors of heme oxygenase, tin protoporphyrin IX and zinc protoporphyrin IX. These compounds are made selective by being administered from a stent where they are present in an amount ranging from 0.1 to 10% by weight of the polymer in the stent and the rate of release is such that duration of drug therapy following implantation is 1 to 20 days or longer.

We turn now to cases where an agent that selectively increases nitrosative stress in pathologically proliferating mammalian cells is present.

In one case of this kind, an agent is administered which activates nitric oxide synthase in mammalian cells. Such agents include cytokines (e.g., interleukin-1, interleukin-2 and interferon-gamma), L-arginine, and calcium ionophors. Selectivity is obtained in application to inhibiting the proliferation of pathologically proliferating cells that would cause restenosis by attachment of the agent to or incorporation of the agent in a polymeric stent implanted during angioplasty with the agent, being present in an amount ranging from 0.1 to 10% by weight of the polymer used in the stent and drug release being such that therapy is continued for 1 to 20 days after implantation of the stent.

In another case of this kind, agent is administered which is a catalytic antibody that makes NO⁻. Examples of these are those acting on anthracene-HNO cycloadduct as described in Bahr, N., et al., J. Am. Chem. Soc. 118, 3550-3555 (1996). Dosages for these are 10 µg to 100 mg/kg body weight per day, and the substrate is given at a dosage of 1 mg to 1 gm/kg body weight per day. Route of administration is local delivery to the pathologically proliferating mammalian cells. Selectivity is obtained by local delivery of the catalytic antibody.

The manipulator of nitrosative stress that is agent that increases nitrosative stress is employed concurrently with a manipulator of nitrosative stress that inhibits protection against nitrosative stress according to the invention. Selectivity is obtained by associating the one manipulator of nitrosative stress that is employed or at least one of a plurality of manipulators of nitrosative stress that are employed with a stent implanted during angioplasty. For example, manipulator(s) of nitrosative stress is (are) attached to a polymeric stent via an ester linkage to polymer of the stent or is (are) associated with a porous polymer coated on a stent whereby manipulator(s) of nitrosative stress leach(es) out of the porous polymer and contact(s) the pathologically proliferating cells. The manipulator(s) of nitrosative stress used in association with a polymeric stent or with polymer coated on a stent is (are) employed in an amount ranging from 0.1 to 10% by weight of the polymer.

Suitable manipulators of nitrosative stress that inhibit protection against nitrosative stress according to the invention include, for example, agents which inhibit the synthesis of glutathione or deplete glutathione non-selectively when not locally delivered, e.g., L-buthionine-S-sulfoximine, diethyl maleate or melphalan. Suitable manipulators of nitrosative stress that increase nitrosative stress include, for example, S-nitrosocysteine, nitroglycerine, amyl nitrite and S-nitroso-polythiodextran. Suitable manipulators of nitrosative stress that inhibit protection against nitrosative stress according to claim 1 for use with manipulators of nitrosative stress that increase nitrosative stress include, for example, melphalan, and thiol synthesis inhibiting agents, e.g., L-buthionine-S-sulfoximine. In one alternative both kinds of manipulators of nitrosative stress are administered from a stent. In other alternatives, L-buthionine-S-sulfoximine is administered from a stent and agent that increases nitrosative stress is administered systemically, e.g., intravenously, or by intracoronary infusion. Where agent that increases nitrosative stress (e.g., S-nitrosocysteine or S-nitrosopolythiodextran) is administered systemically, it is preferably used in a dosage ranging from 0.1 µg to 100 mg per kg mammalian body weight per day and administration is preferably by oral or intravenous administration. The treatments are optionally used in combination with intravascular beta-, gamma- or X-ray irradiation. Methods for applying beta- and gamma-irradiation for this purpose are described hereinbefore.

Still another embodiment herein uses a pathologically proliferating mammalian cell antiproliferative effective amount of a manipulator of nitrosative stress comprising agent functional to convert endogenously produced NO to NO⁻ or NO⁺ (including species with NO⁺ activity which are nitric-oxide related compounds). Example of agent that is functional to convert endogenously produced NO to NO⁻ or NO⁺ is redox active metal catalyst, especially chelates of iron which are known to be redox active metal catalysts. An example of a redox active metal catalyst that is functional to convert endogenously produced NO to NO⁻ is ferrioxamine B complex which is referred to as [Fe(III) (HDFB)]⁺ and as a stable and efficient electrocatalyst for the reduction of NO to NO₂ at biologically relevant potentials in Kazmierski, W. M., et al., Proc. Natl. Acad. Sci. USA, Vol. 93, pp 9138-9141 (8/1996). Redox active metal catalysts functional to convert NO to NO⁺ are described in Stamler, J., et al., Science, Vol. 258, 1898-1902 (1992). Redox active catalysts functional to convert NO to species with NO⁺ activity are heme complexes including myoglobin and hemoglobin. Preferably cytokines are also administered which induce iNOS to cause increase of endogenous NO production resulting in higher levels of nitrosative stress because of greater efficiency of conversion to nitrosative stress species because of the greater amount of NO being produced. Cytokines useful in this embodiment to induce iNOS to cause increased endogenous NO production include interleukin-1, interleukin-2 and tumor necrosis factor. Dosages for the cytokines are 1 µg to 10 mg/kg body weight per day for 1 to 10 days and route of administration is intravenous.

The invention herein is illustrated by the following examples.

### EXAMPLE I

A 55 year old white male smoker presents with chest pain. His coronary angiogram shows 99% left anterior descending coronary artery stenosis. The patient is treated with 80 mg verapamil (a blocker of the ability of the cells to export nitrosant three times a day) p.o. and with L-buthionine-S-sulfoximine (an inhibitor of protection against nitrosative stress), i.e., BSO (4 mmol/kg i.v. four times a day). A stent constructed of a NO-releasing polymer is placed in the constricted artery. The patient rapidly becomes pain free and a repeat angiogram at six months shows no restenosis.

A patient similar to the one described above is treated with a BSO-releasing polymer stent and is given S-nitrosocysteine (a compound that increases nitrosative stress) (100 nmol/kg body weight over one min.) by intracoronary infusion. The patient reports no chest pain and a follow-up angiogram shows no restenosis.

### EXAMPLE II

A 45 year old black male with blue eyes and gray hair presents with chest pain. His coronary angiogram shows an 80% occlusion of his left anterior descending coronary artery before the first diagonal. The patient undergoes balloon angioplasty and placement of a Palmaz-Schatz stent, which is P³²-implanted and coated with a cyclodextran polymer to which BSO is attached by an ester linkage. The patient's symptoms resolve and clinical restenosis does not occur.

A patient similar to the one described above undergoes balloon angioplasty and placement of a Palmaz-Schatz stent which is coated with cyclodextran polymer to which ethyl maleate (a depleter of glutathione) is attached by an ester linkage. The patient's symptoms resolve and clinical restenosis does not occur.

### EXAMPLE III

A 65 year old white male with three-vessel disease status post cabbage presents with chest pain. His angiogram shows that his saphenous vein graft to the right coronary artery has closed. A Palmaz-Schatz stent coated with an ONO-substituted polymer to which melphalan (an inhibitor of protection against nitrosative stress) has been chemically attached in an amount of 10% by weight of the polymer is placed. The patient's symptoms resolve and clinical restenosis does not occur.

Variations of the invention will be obvious to those skilled in the art. Therefore the invention is defined by the claims.

## Claims

1. A stent comprising a manipulator of nitrosative stress which inhibits protection against nitrosative stress by downregulating nitrosative stress defense mechanism in pathologically proliferating mammalian cells to selectively kill or reduce the growth of pathologically proliferating cells that would cause restenosis, said manipulator being selected from:.
L-buthionine-S-sulfoximine **("BSO")**,
ethyl maleate,
diethyl maleate,
melphalan,
an aminotriazole,
tin protoporphyrin IX, and
zinc protoporphyrin IX.

2. The use of a manipulator of nitrosative stress which inhibits protection against nitrosative stress by downregulating nitrosative stress defense mechanism in pathologically proliferating mammalian cells to selectively kill or reduce the growth of pathologically proliferating cells in the manufacture of a medicament comprised in a stent for the prevention of restenosis, said manipulator being selected from:.
L-buthionine-S-sulfoximine **("BSO")**,
ethyl maleate,
diethyl maleate,
melphalan,
an aminotriazole,
tin protoporphyrin IX, and
zinc protoporphyrin IX.

3. A stent of Claim 1 or use of Claim 2, wherein the manipulator is attached to a polymer stent.

4. A stent of Claim 1 or use of Claim 2, wherein the manipulator is incorporated in a porous polymer stent.

5. A stent of Claim 1 or use of Claim 2, wherein the manipulator is attached to or incorporated in a polymer coating on the stent.

6. A stent of Claim 1 or use of Claim 2, wherein the manipulator is associated with a porous polymer coated on the stent whereby the manipulator leaches out of the porous polymer and contacts the pathologically proliferating cells.

7. A stent of Claim 1 or use of Claim 2, wherein one or more inhibitors of protection against nitrosative stress are the only kind of manipulator of nitrosative stress comprised in the stent.

8. A stent of Claim 1 or use of Claim 2, wherein the stent also comprises a manipulator of nitrosative stress which increases nitrosative stress

## Patentansprüche

1. Stent aufweisend ein Manipulierungsmittel für nitrosativen Stress, das den Schutz gegen nitrosativen Stress durch Herunterregulieren des Abwehrmechanismus gegen nitrosativen Stress bei pathologisch proliferierenden Säugetierzellen hemmt, um pathologisch proliferierende Zellen, die Restenose verursachen würden, selektiv abzutöten oder ihr Wachstum selektiv zu verringern, wobei das Manipulierungsmittel ausgewählt ist aus:
L-Buthionin-S-sulfoximin ("**BSO**"),
Ethylmaleat,
Diethylmaleat,
Melphalan,
einem Aminotriazol,
Zinn-Protoporphyrin IX, und
Zink-Protoporphyrin IX.

2. Verwendung eines Manipulierungsmittels für nitrosativen Stress, das den Schutz gegen nitrosativen Stress durch Herunterregulieren des Abwehrmechanismus gegen nitrosativen Stress bei pathologisch proliferierenden Säugetierzellen hemmt, um pathologisch proliferierende Zellen selektiv abzutöten oder ihr Wachstum selektiv zu verringern, bei der Herstellung eines in einem Stent enthaltenen Arzneimittels zur Verhinderung von Restenose, wobei das Manipulierungsmittel ausgewählt wird aus:
L-Buthionin-S-sulfoximin ("**BSO**"),
Ethylmaleat,
Diethylmaleat,
Melphalan,
einem Aminotriazol,
Zinn-Protoporphyrin IX, und
Zink-Protoporphyrin IX.

3. Stent nach Anspruch 1 oder Verwendung nach Anspruch 2, wobei das Manipulierungsmittel an einem Polymer-Stent angebracht ist.

4. Stent nach Anspruch 1 oder Verwendung nach Anspruch 2, wobei das Manipulierungsmittel in einen porösen Polymer-Stent inkorporiert ist.

5. Stent nach Anspruch 1 oder Verwendung nach Anspruch 2, wobei das Manipulierungsmittel an einer Polymer-Beschichtung auf dem Stent angebracht oder in eine Polymer-beschichtung auf dem Stent inkorporiert ist.

6. Stent nach Anspruch 1 oder Verwendung nach Anspruch 2, wobei das Manipulierungsmittel vereint mit einem porösen Polymer, das auf den Stent aufgetragen ist, vorliegt, wodurch das Manipulierungsmittel aus dem porösen Polymer austritt und mit den pathologisch proliferierenden Zellen in Kontakt kommt.

7. Stent nach Anspruch 1 oder Verwendung nach Anspruch 2, wobei ein Hemmstoff oder mehrere Hemmstoffe des Schutzes gegen nitrosativen Stress die einzige Art von Manipulierungsmittel für nitrosativen Stress, das in dem Stent enthalten ist, ist (sind).

8. Stent nach Anspruch 1 oder Verwendung nach Anspruch 2, wobei der Stent auch ein Manipulierungsmittel für nitrosativen Stress, das den nitrosativen Stress erhöht, aufweist.

## Revendications

1. Endoprothèse comprenant un manipulateur du stress nitrosant qui inhibe la protection contre le stress nitrosant en régulant à la baisse le mécanisme de défense contre le stress nitrosant dans des cellules de mammifère à prolifération pathologique pour tuer de façon sélective ou réduire la croissance des celles à prolifération pathologique qui entraîneraient une resténose, ledit manipulateur étant choisi parmi :
une L-buthionine-S-sulfoximine (« BSO »)
le maléate d'éthyle
le maléate de diéthyle
le melphalan
un aminotriazole
la protoporphyrine d'étain IX, et
la protoporphyrine de zinc IX.

2. Utilisation d'un manipulateur du stress nitrosant qui inhibe la protection contre le stress nitrosant en régulant à la baisse le mécanisme de défense contre le stress nitrosant dans des cellules de mammifère à prolifération pathologique pour tuer de façon sélective ou réduire la croissance des celles à prolifération pathologique dans la fabrication d'un médicament compris dans une endoprothèse pour la prévention de la resténose, ledit manipulateur étant choisi parmi :
une L-buthionine-S-sulfoximine (« BSO »)
le maléate d'éthyle
le maléate de diéthyle
le melphalan
un aminotriazole
la protoporphyrine d'étain IX, et
la protoporphyrine de zinc IX.

3. Endoprothèse selon la revendication 1 ou utilisation selon la revendication 2, dans laquelle le manipulateur est fixé à une endoprothèse en polymère.

4. Endoprothèse selon la revendication 1 ou utilisation selon la revendication 2, dans laquelle le manipulateur est incorporé dans une endoprothèse en polymère poreux.

5. Endoprothèse selon la revendication 1 ou utilisation selon la revendication 2, dans laquelle le manipulateur est fixé à ou incorporé dans un revêtement polymère sur l'endoprothèse.

6. Endoprothèse selon la revendication 1 ou utilisation selon la revendication 2, dans laquelle le manipulateur est associé à un polymère poreux enduit sur l'endoprothèse, ce par quoi le manipulateur s'extrait du polymère poreux et est en contact avec les cellules à prolifération pathologique.

7. Endoprothèse selon la revendication 1 ou utilisation selon la revendication 2, dans laquelle un ou plusieurs inhibiteur(s) de la protection contre le stress nitrosant est/sont le seul type de manipulateur du stress nitrosant compris dans l'endoprothèse.

8. Endoprothèse selon la revendication 1 ou utilisation selon la revendication 2, dans laquelle l'endoprothèse comprend également un manipulateur du stress nitrosant qui augmente le stress nitrosant.
